# EUROPEAN PATENT APPLICATION

(11) **EP 4 752 677 A1**
(43) Date of publication of application: **03.06.2026**
(21) Application number: 24865671.2
(22) Date of filing: 25.07.2024
(51) Int. Cl.: G06F 1/16, G06F 11/32, G02B 6/00, F21K 9/61, G06F 3/01, F21Y 115/10

(54) **WEARABLE ELECTRONIC DEVICE COMPRISING LIGHT EMITTING UNIT**

(30) Priority: 12.09.2023 KR 20230121316; 06.10.2023 KR 20230133585
(71) Applicant: Samsung Electronics Co., Ltd., Suwon-si, Gyeonggi-do 16677 (KR)
(72) Inventor: JUNG, Hyunjun, Suwon-si, Gyeonggi-do 16677 (KR); KWON, Hyoujoo, Suwon-si, Gyeonggi-do 16677 (KR); PARK, Hyuncheol, Suwon-si, Gyeonggi-do 16677 (KR); YOO, Soohan, Suwon-si, Gyeonggi-do 16677 (KR); LEE, Seungwon, Suwon-si, Gyeonggi-do 16677 (KR); JO, Seongwook, Suwon-si, Gyeonggi-do 16677 (KR)
(74) Representative: Gulde & Partner
(86) International application number: PCT/KR2024/010825
(87) International publication number: WO 2025/058228

(57) **Abstract**

According to an embodiment of the present disclosure, a wearable electronic device may comprise: a housing; and a substrate arranged in an inner space of the housing. According to an embodiment, the wearable electronic device may comprise at least one light emitting unit arranged on the substrate. According to an embodiment, the wearable electronic device may include at least one light guide member arranged adjacent to each of the at least one light emitting unit when the wearable electronic device is viewed from one side. According to an embodiment, the wearable electronic device may comprise at least one light scattering member arranged such that light emitted through the at least one light emitting unit and reflected by the at least one light guide member is transmitted to the outside. In addition to various embodiments disclosed in the present document, various other embodiments are possible.

## Description

### [Technical Field]

Embodiments disclosed herein relate to a wearable electronic device including a light emitting unit.

### [Background Art]

An electronic device may include a wearable electronic device that is configured to be worn on a part of a user's body so as to improve portability or user accessibility. The wearable electronic device may include a ring type wearable electronic device that is worn on a user's finger and provides various user experiences and beneficial functions. The ring type wearable electronic device may include various sensors capable of measuring biometric information of the user. For example, as one of the various sensors, the wearable electronic device may include an optical sensor including at least one light emitting unit and a light receiving unit.

The above-described information may be provided as related art for the purpose of assisting in understanding the disclosure. No assertion or decision is made as to whether any of the above might be applicable as prior art with regard to the disclosure.

### [Disclosure of Invention]

### [Technical Problem]

A ring type wearable electronic device is a small-sized electronic device, and a layout design of an indicator, such as an LED, capable of providing or outputting visual notification information to a user in the small-sized electronic device may be required.

According to an embodiment of the disclosure, the wearable electronic device may include an optical sensor provided to measure biometric information of a user and at least one light structure additionally disposed. The wearable electronic device may allow light emitted through at least one light emitting unit of the optical sensor and reflected by the at least one light structure to be emitted to the outside.

### [Solution to Problem]

According to an embodiment of the disclosure, a wearable electronic device may include a housing and a substrate disposed in an inner space of the housing. According to an embodiment, the wearable electronic device may include at least one light emitting unit disposed on the substrate. According to an embodiment, when the wearable electronic device is viewed from one side, the wearable electronic device may include at least one light guide member disposed adjacent to the at least one light emitting unit. According to an embodiment, the wearable electronic device may include at least one light scattering member disposed such that light emitted through the at least one light emitting unit and reflected by the at least one light guide member is transmitted to the outside.

According to an embodiment of the disclosure, a wearable electronic device may include a housing and a substrate disposed in an inner space of the housing. According to an embodiment, the wearable electronic device may include at least one light emitting unit disposed on the substrate. According to an embodiment, when the wearable electronic device is viewed from the front, the wearable electronic device may include at least one light scattering member disposed in the inner space of the housing so as to face the at least one light emitting unit. According to an embodiment, light emitted through the at least one light emitting unit may be reflected by the at least one light scattering member and transmitted to the outside.

### [Advantageous Effects of Invention]

According to an embodiment of the disclosure, the wearable electronic device may allow a user to intuitively recognize visual notification information related to an event of the wearable electronic device, as light emitted through at least one light emitting unit of an optical sensor and reflected by at least one light structure is transmitted to the outside.

According to an embodiment of the disclosure, the wearable electronic device may provide visual notification information by using a light emitting unit of an optical sensor provided to measure biometric information, thereby securing efficient mounting space and contributing to a slimming of the wearable electronic device.

### [Brief Description of Drawings]

FIG. 1 is a block diagram of an electronic device in a network environment according to an embodiment of the disclosure.
FIG. 2 is a cross-sectional view illustrating a wearable electronic device, viewed from the front, according to an embodiment of the disclosure.
FIG. 3A is a cross-sectional view illustrating a wearable electronic device including a light emitting unit, viewed from the front, according to an embodiment of the disclosure.
FIG. 3B is a view illustrating a wearable electronic device including a light emitting unit, viewed from one side, according to an embodiment of the disclosure.
FIG. 4A is a view illustrating a wearable electronic device including a plurality of light emitting units, according to an embodiment of the disclosure.
FIG. 4B is a view illustrating a wearable electronic device including a plurality of light emitting units, according to an embodiment of the disclosure.
FIG. 5 is a cross-sectional view illustrating a wearable electronic device including a plurality of light emitting units, viewed from the front, according to an embodiment of the disclosure.
FIG. 6A is a cross-sectional view illustrating a wearable electronic device including a light emitting unit, viewed from the front, according to an embodiment of the disclosure.
FIG. 6B is a view illustrating a wearable electronic device including a light emitting unit, viewed from one side, according to an embodiment of the disclosure.
FIG. 7 is a cross-sectional view illustrating a wearable electronic device including a plurality of light emitting units, viewed from the front, according to an embodiment of the disclosure.
FIG. 8 is a view illustrating modes in which a plurality of light emitting units are disposed in a wearable electronic device, according to an embodiment of the disclosure.

### [Mode for the Invention]

Hereinafter, embodiments of the disclosure will be described in detail with reference to the accompanying drawings so that those skilled in the art to which the disclosure pertains can easily implement the disclosure. However, the disclosure may be implemented in various different forms and is not limited to embodiments set forth herein. With regard to the description of the drawings, the same or like reference signs may be used to designate the same or like elements. Also, in the drawings and the relevant descriptions, description of well-known functions and configurations may be omitted for the sake of clarity and brevity.

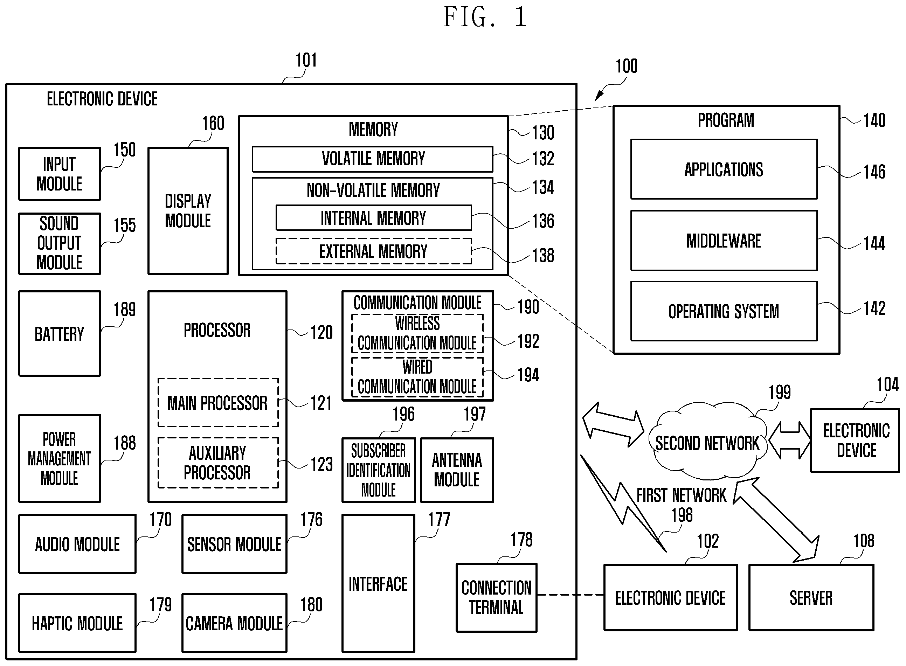

FIG. 1 is a block diagram illustrating an electronic device 101 in a network environment 100 according to various embodiments.

Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connection terminal 178, a haptic module 179, a camera module 180, a power management module 188, a battery 189, a communication module 190, a subscriber identification module (SIM) 196, or an antenna module 197. In some embodiments, at least one of the components (e.g., the connection terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134. The non-volatile memory 134 may include an internal memory 136 and/or an external memory 138.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) (e.g., speaker or headphone) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., through wires) or wirelessly. According to an embodiment, the interface 177 may include, for example, a high-definition multimedia interface (HDMI), a universal serial bus (USB) interface, a secure digital (SD) card interface, or an audio interface.

The connection terminal 178 may include a connector via which the electronic device 101 may be physically connected with the external electronic device (e.g., the electronic device 102). According to an embodiment, the connection terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., an application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, Wi-Fi direct, or infrared data association (IrDA)) or the second network 199 (e.g., a long-range communication network, such as a legacy cellular network, a fifth generation (5G) network, a next-generation communication network, the Internet, or a computer network (e.g., LAN or wide area network (WAN))). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large-scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., an mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

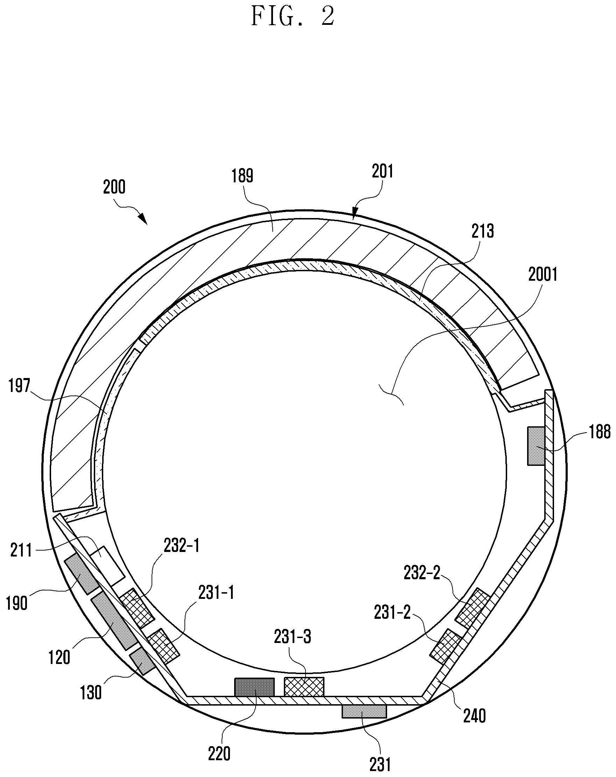

FIG. 2 is a cross-sectional view of a wearable electronic device viewed from the front according to an embodiment of the disclosure.

The wearable electronic device 200 in FIG. 2 may be at least partially similar to the electronic device 101 in FIG. 1 or further include other embodiments of the electronic device. For example, the wearable electronic device 200 may include a smart ring capable of being worn on a part of a human body (e.g., a finger). However, the disclosure is not limited thereto.

In the description of the wearable electronic device 200 according to the disclosure, a ring-type (e.g., ring-shape) wearable electronic device worn on a user's finger is illustrated and described. However, the disclosure is not limited thereto. For example, the wearable electronic device 200 may include a bracelet-type wearable electronic device, an open-ring-type electronic device with a portion open, or a curved or non-curved electronic device.

With reference to FIG. 2, the wearable electronic device 200 may include an annular housing 201 including an opening 2001 therein. In an embodiment, the housing 201 may include a first surface (e.g., an outer surface or an outer ring housing) configured to be exposed to an external environment in a state in which the wearable electronic device 200 is worn on a part of the human body (e.g., the finger), and a second surface (e.g., an inner surface or an inner ring housing) positioned to be opposite to the first surface and configured to be at least partially in contact with the finger's skin in the worn state. For example, the outer surface of the housing (201) may correspond to a large circular circumference, and the inner surface may correspond to a small circular circumference.

In an embodiment, the opening 2001 may be sized so that the user's finger is fitted with the opening 2001.

In an embodiment, the wearable electronic device 200 may include a battery 189 and a substrate 240 disposed within an inner space of the housing 201. In an embodiment, the battery 189 and the substrate 240 may be disposed in opposite directions. However, the disclosure is not limited thereto.

In an embodiment, the substrate 240 may be disposed such that it is attached to an inner surface of the housing 201. For example, the substrate 240 may include a flexible printed circuit board (FPCB). For example, the substrate 240 may have a bendability to correspond to a curvature of the wearable electronic device 200 (e.g., an inner curvature corresponding to an opening 2001 of the housing 201). In some embodiments, the substrate 240 may include a substrate or a plurality of hard type substrates (PCBs, printed circuit boards) that include a hard type area having a width and length that are not affected by the curvature of the housing 201.

With reference to FIG. 2, a plurality of electric elements substrate 240 may disposed on the substrate 240. In an embodiment, the plurality of electric elements may include at least one constituent element among at least one biometric sensor (e.g., temperature sensors 210 and 211 and/or PPG sensors 231-1, 231-2, 231-3, 232-1, and 232-2) disposed to detect (or acquire) bio-information (e.g., a body temperature) of a user through at least a part (e.g., the first surface or the second surface) of the housing 201, an inertial sensor 220 (e.g., a 3-axis sensor, a 6-axis sensor, an acceleration sensor, or a gyro sensor) configured to detect a motion of the wearable electronic device 200, a temperature sensor 211, a processor (e.g., the processor 120 of FIG. 1), a memory (e.g., the memory 130 of FIG. 1), a communication circuit (e.g., the communication module 190 of FIG. 1), and/or a power management module (e.g., the power management module 188 of FIG

According to an embodiment, at least one biometric sensor (e.g., PPG sensors 231-1, 231-2, 231-3, 232-1, and 232-2) (e.g., a photoplethysmogram sensor or a photoplethysmography sensor) may acquire bio-information of the user.

According to an embodiment, the PPG sensors 231-1, 231-2, 231-3, 232-1, and 232-2 may measure pulse waves of the user positioned in a second direction, along the second surface (e.g., the inner surface) of the housing 201. For example, the PPG sensors may include light-emitting-PPG sensors 231-1, 231-2, and 231-3 configured to emit light, and light-receiving-PPG sensors 232-1 and 232-2 configured to receive light.

According to the embodiment, the PPG sensors 231-1, 231-2, 231-3, 232-1, and 232-2 (e.g., the photoplethysmogram sensor or the photoplethysmography sensor) disposed on the second surface (e.g., the inner surface) of the housing 201 may identify a flow of blood (e.g., a blood flow) by using light. For example, when the blood flows along blood vessels, a blood flow rate finely changes. The PPG sensors may measure the pulse waves (plethysmogram (PTG)) of the user (e.g., the user wearing the wearable electronic device 200 on the finger) on the basis of the amount of change in blood flow rates. The PPG sensors may include the light-emitting-PPG sensors 231-1, 231-2, and 231-3 configured to emit light, and the light-receiving-PPG sensors 232-1 and 232-2 configured to receive light. For example, the light emitted from the light-emitting-PPG sensors 231-1, 231-2, and 231-3 is at least partially reflected by the object, and the light-receiving-PPG sensors 232-1 and 232-2 may receive at least a part of the reflected light. The processor 120 may measure the user's pulse waves on the basis of the received light. According to an embodiment, the PPG sensors 231-1, 231-2, 231-3, 232-1, and 232-2 may be controlled by a PPG control module 231. According to an embodiment, the PPG sensor is not limited to a particular type (e.g., a reflective type or a transmissive type) and to a blood flow measurement method based on a particular method. For example, the PPG sensor may be implemented as a reflective type, a transmissive type, and other types.

In an embodiment, the temperature sensor 211 may measure the user's body temperature. However, the disclosure is not limited thereto. For example, the temperature sensor (211) may measure the temperature of at least one of a plurality of electric elements included in the wearable electronic device 200.

In an embodiment, when the occurrence of an event is detected, the processor 120 may emit light through the light-emitting-PPG sensors 231-1, 231-2, and 231-3 that emit light from the PPG sensors, thereby providing (e.g., outputting) visual notification information related to the event.

According to an embodiment, an inertial sensor 220 may include at least one of a 3-axis sensor, a 6-axis sensor, an acceleration sensor, or a gyro sensor. According to an embodiment, the inertial sensor 220 may determine an arrangement angle, an arrangement posture, and an arrangement position of the inertial sensor 220 based on a preset particular posture (e.g., an upright posture) of the wearable electronic device 200. The inertial sensor 220 may acquire coordinate information (e.g., gesture information or movement information) in accordance with a posture, a position, and/or a motion of the wearable electronic device 200. According to an embodiment, the processor 120 may detect various motions of the wearable electronic device 200 (e.g., finger movements of the user wearing the wearable electronic device 200) based on the inertial sensor 220.

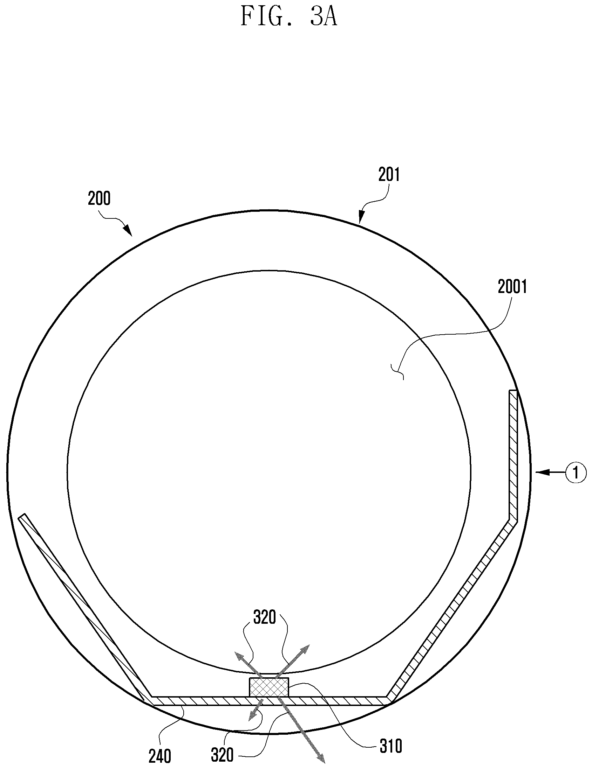

According to an embodiment, the power management module (e.g., the power management module 188 of FIG. 1) may manage electric power supplied to at least one electric element included in the wearable electronic device 200. For example, the power management module 188 may use a charging interface 213 to manage electric power supplied to the constituent elements from a battery (e.g., the battery 189 or FIG. 1). For example, the power management module 188 may include at least a part of a power management integrated circuit (PMIC). In an embodiment, the battery 189 may supply power to the substrate 240, and the supplied power can be used to light a light emitting unit (e.g., light emitting unit 310 of FIG. 3A).

According to an embodiment, the communication module (e.g., the communication module 190 in FIG. 1) may connect the wearable electronic device 200 and an external electronic device (e.g., the electronic devices 102 and 104 in FIG. 1). For example, the communication module 190 may be electrically connected to an antenna module (e.g., the antenna module 197 in FIG. 1) and transmit signals or electric power to the external electronic devices 102 and 104 or receive signals or electric power from the external electronic devices 102 and 104 through the antenna module 197. For example, the antenna module 197 may include a conductor formed on the substrate 240 or a radiator configured by a conductive pattern.

According to an embodiment, the wearable electronic device 200 may include at least one of at least one display (not illustrated) (e.g., the display module 160 of FIG. 1) configured to provide the user with visual output information, an audio module (e.g., the audio module 170 or FIG. 1) configured to provide the user with auditory output information, and/or a haptic module (not illustrated) (e.g., the haptic module 179 of FIG. 1) configured to provide the user with tactile output information.

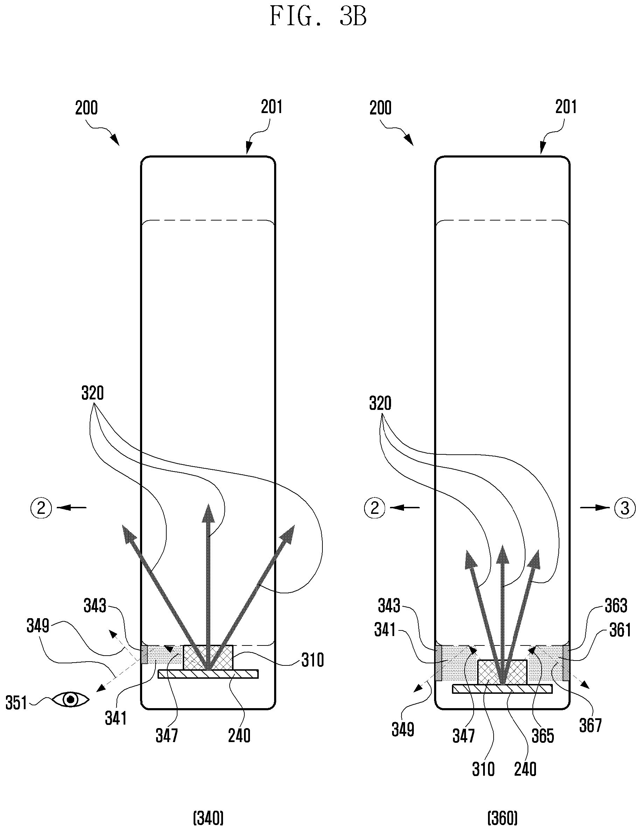

FIG. 3A is a cross-sectional view illustrating, when viewed from the front, a wearable electronic device 200 including a light emitting unit 310 according to an embodiment of the disclosure. FIG. 3B is a view illustrating, when viewed from one side, a wearable electronic device 200 including a light emitting unit 310 according to an embodiment of the disclosure.

Referring to FIGS. 3A and 3B, a wearable electronic device (e.g., the wearable electronic device 200 of FIG. 2) may include a light emitting unit 310 (e.g., the light emitting PPG sensors 231-1, 231-2, and 231-3 of FIG. 2).

According to an embodiment, the wearable electronic device 200 may include a substrate 240 disposed in an inner space of a housing 201. For example, the substrate 240 may have flexibility so as to correspond to a curvature of the wearable electronic device 200 (e.g., an inner curvature corresponding to an opening 2001 of the housing 201).

According to an embodiment, the light emitting unit 310 may be disposed on the substrate 240. For example, the light emitting unit 310 may be disposed on the substrate 240 so as to face a specific direction (e.g., to be directed toward the opening 2001).

According to an embodiment, the light emitting unit 310 may include a light emitting diode (LED).

Referring to <340> of FIG. 3B according to an embodiment, the wearable electronic device 200 may include at least one light guide member disposed adjacent to a light emitting unit 310 when the wearable electronic device 200 is viewed from one side.

According to an embodiment, the at least one light guide member may include a first light guide member 341. According to an embodiment, when the wearable electronic device 200 is viewed from one side (e.g., direction ① of FIG. 3A), the first light guide member 341 may be disposed adjacent to the light emitting unit 310 in a first lateral direction (e.g., direction ②) from the light emitting unit 310.

According to an embodiment, the first light guide member 341 may include a total reflection film optical fiber. However, the disclosure is not limited thereto.

According to an embodiment, the wearable electronic device 200 may include at least one light scattering member disposed such that light emitted through the light emitting unit 310 and reflected (or collected) by at least one light guide member (e.g., the first light guide member 341) is transmitted (e.g., scattered) to the outside.

According to an embodiment, the at least one light scattering member may include a first light scattering member 343. According to an embodiment, when the wearable electronic device 200 is viewed from the one side (e.g., in direction ① of FIG. 3A), the first light scattering member 343 may be disposed on at least a portion of the housing 201 in the first lateral direction (e.g., direction ②) from the first light guide member 341.

According to an embodiment, the first light scattering member 343 may include a light diffusion film. However, the disclosure is not limited thereto.

According to an embodiment, at least a portion of light 347 from light 320 and 347 having a wide emission angle emitted through the light emitting unit 310 may be reflected (or collected) by the first light guide member 341. The light 347 reflected (or collected) by the first light guide member 341 may be scattered 349 by the first light scattering member 343 and transmitted to the outside. As the light 347 reflected by the first light guide member 341 is scattered (349) by the first light scattering member 343 and transmitted to the outside, the scattered light 349 may be recognized through a user's eyes 351.

Referring to <360> of FIG. 3B according to an embodiment, the at least one light guide member may include the first light guide member 341 and a second light guide member 361.

According to an embodiment, when the wearable electronic device 200 is viewed from the one side (e.g., direction ① of FIG. 3A), the first light guide member 341 may be disposed adjacent to the light emitting unit 310 in the first lateral direction (e.g., direction ②) from the light emitting unit 310. According to an embodiment, when the wearable electronic device 200 is viewed from the one side (e.g., in direction ① of FIG. 3A), the second light guide member 361 may be disposed adjacent to the light emitting unit 310 in a second lateral direction (e.g., direction ③) of the light emitting unit 310.

According to an embodiment, the first light guide member 341 and/or the second light guide member 361 may include a total reflection film optical fiber. However, the disclosure is not limited thereto.

According to an embodiment, the wearable electronic device 200 may include at least one light scattering member disposed such that light emitted through the light emitting unit 310 and reflected (or collected) by the at least one light guide member (e.g., the first light guide member 341 and/or the second light guide member 361) is transmitted to the outside (e.g., scattered).

According to an embodiment, the at least one light scattering member may include a first light scattering member 343 and a second light scattering member 363.

According to an embodiment, when the wearable electronic device 200 is viewed from the one side (e.g., in direction ① of FIG. 3A), the first light scattering member 343 may be disposed on at least a portion of the housing 201 in the first lateral direction (e.g., direction ②) from the first light guide member 341. According to an embodiment, when the wearable electronic device 200 is viewed from the one side (e.g., in direction ① of FIG. 3A), the second light scattering member 363 may be disposed on at least a portion of the housing 201 in the second lateral direction (e.g., direction ③) from the second light guide member 361.

According to an embodiment, the first light scattering member 343 and/or the second light scattering member 363 may include a light diffusion film. However, the disclosure is not limited thereto.

According to an embodiment, from light 320, 347, and 365 having a wide emission angle emitted through the light emitting unit 310, at least a portion of the light 347 may be reflected (or collected) by the first light guide member 341, and at least a portion of other light 365 may be reflected (or collected) by the second light guide member 361. The light 347 reflected (or collected) by the first light guide member 341 may be scattered (349) by the first light scattering member 343 and transmitted to the outside, and the light 365 reflected (or collected) by the second light guide member 361 may be scattered (367) by the second light scattering member 363 and transmitted to the outside. As the light 347 reflected (or collected) by the first light guide member 341 and the light 365 reflected (or collected) by the second light guide member 361 are scattered (349, 367) by the first light scattering member 343 and the second light scattering member 363 and transmitted to the outside, the scattered light 349 and 367 may be recognized through a user's eye 351.

In various embodiments, the light emitting unit 310 may emit light 320, 347, and/or 365 under the control of a processor of the wearable electronic device 200 (e.g., the processor 120 of FIG. 1) when an occurrence of an event is detected. As described above with reference to FIGS. 3A and 3B, as an occurrence of an event is detected, light 320, 347, and/or 365 emitted through the light emitting unit 310 may be reflected (or collected) by a light guide member (e.g., the first light guide member 341 and/or the second light guide member 361). As the light 347 and/or 365 reflected (or collected) by the light guide member is scattered (349 and/or 367) by a light scattering member (e.g., the first light scattering member 343 and/or the second light scattering member 363) and transmitted to the outside, the light may be recognized through a user's eye 351, such that the user may intuitively identify visual notification information related to the event.

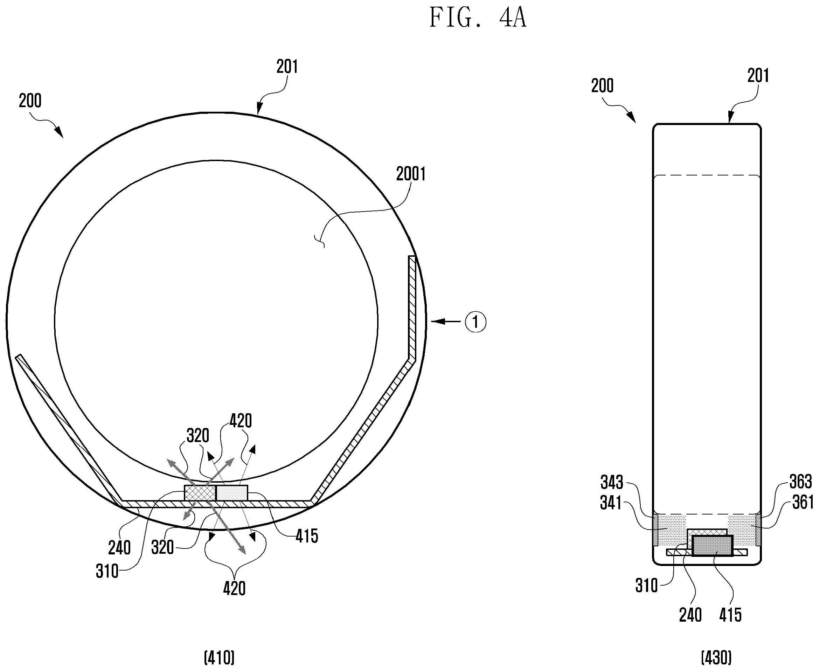

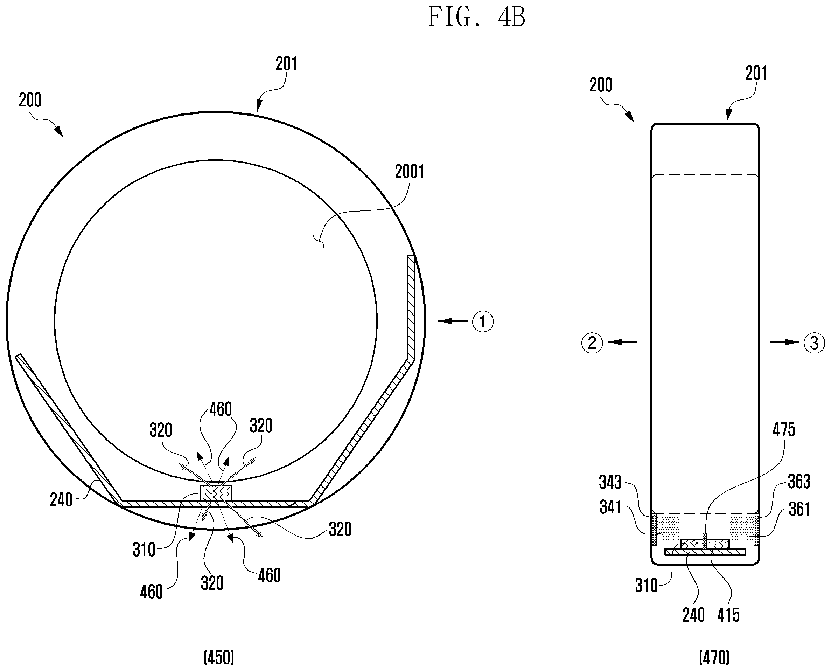

In FIGS. 3A and 3B according to various embodiments, the wearable electronic device 200 has been described as including a single light emitting unit (e.g., the light emitting unit 310); however, the disclosure is not limited thereto. For example, the wearable electronic device 200 may include a plurality of light emitting units. In this regard, various embodiments will be described below with reference to FIGS. 4A and 4B.

FIG. 4A is a view illustrating a wearable electronic device 200 including a plurality of light emitting units 310 and 415 according to an embodiment of the disclosure.

According to an embodiment, <410> of FIG. 4A is a cross-sectional view illustrating, when viewed from the front, a wearable electronic device (e.g., the wearable electronic device 200 of FIG. 2) including a plurality of light emitting units (e.g., the first light emitting unit 310 and the second light emitting unit 415). According to an embodiment, <430> of FIG. 4A is a view illustrating, when viewed from one side (e.g., in direction ①), a wearable electronic device 200 including a plurality of light emitting units 310 and 415.

Referring to <410> and <430> of FIG. 4A, the wearable electronic device 200 may include a plurality of light emitting units (e.g., the first light emitting unit 310 and the second light emitting unit 415). For example, the plurality of light emitting units (e.g., the first light emitting unit 310 and the second light emitting unit 415) may be disposed on a substrate 240 so as to face a specific direction (e.g., to be directed toward the opening 2001). For example, when the wearable electronic device 200 is viewed from the front, the plurality of light emitting units (e.g., the first light emitting unit 310 and the second light emitting unit 415) may be disposed adjacent to each other (e.g., arranged side by side) on the substrate 240. As another example, when the wearable electronic device 200 is viewed from one side (e.g., in direction ①), the plurality of light emitting units (e.g., the first light emitting unit 310 and the second light emitting unit 415) may be disposed on the substrate 240 so as to overlap each other.

According to an embodiment, a plurality of light emitting units (e.g., the first light emitting unit 310 and the second light emitting unit 415) may have different wavelength bands.

According to an embodiment, the plurality of light emitting units (e.g., the first light emitting unit 310 and/or the second light emitting unit 415) may emit light (e.g., first light 320 and/or second light 420) under the control of a processor of the wearable electronic device 200 (e.g., the processor 120 of FIG. 1) when an occurrence of an event is detected. For example, when an occurrence of an event is detected, the first light emitting unit 310 may emit first light 320 of a first wavelength band (e.g., a green wavelength band) under the control of the processor 120. When an occurrence of an event is detected, the second light emitting unit 415 may emit second light 420 of a second wavelength band (e.g., a red wavelength band) under the control of the processor 120.

According to an embodiment, when light (e.g., the first light 320 and/or the second light 420) is emitted through the first light emitting unit 310 and the second light emitting unit 415 having different wavelength bands, a new wavelength band may be additionally implemented. For example, when the first light 320 of the first wavelength band (e.g., the green wavelength band) is emitted through the first light emitting unit 310 and the second light 420 of the second wavelength band (e.g., the red wavelength band) is emitted through the second light emitting unit 415, light of a new wavelength band (e.g., a third wavelength band (e.g., a yellow wavelength band)) may be implemented.

In various embodiments, the first light emitting unit 310 and/or the second light emitting unit 415 may emit light (e.g., first light 320 and/or second light 420) under the control of a processor of the wearable electronic device 200 (e.g., the processor 120 of FIG. 1) when an occurrence of an event is detected. As an occurrence of an event is detected, the first light 320 emitted through the first light emitting unit 310 may be reflected (or collected) by a light guide member (e.g., the first light guide member 341 and/or the second light guide member 361). In addition, as an occurrence of an event is detected, the second light 420 emitted through the second light emitting unit 415 may be reflected (or collected) by a light guide member (e.g., the first light guide member 341 and/or the second light guide member 361). As the light 320 and/or 420 reflected (or collected) by the light guide member is scattered by a light scattering member (e.g., the first light scattering member 343 and/or the second light scattering member 363) and transmitted to the outside, the light may be recognized through a user's eye (e.g., 351 of FIG. 3B), such that the user may intuitively identify visual notification information related to the event.

In <430> of FIG. 4A according to various embodiments, when the wearable electronic device 200 is viewed from one side (e.g., in direction ①), the first light emitting unit 310 disposed at a rear side of the second light emitting unit 415 is illustrated as being visible; however, the plurality of light emitting units (e.g., the first light emitting unit 310 and the second light emitting unit 415) may be disposed to overlap each other, such that, when the wearable electronic device 200 is viewed from one side (e.g., in direction ①), the first light emitting unit 310 disposed at the rear side of the second light emitting unit 415 may be invisible.

In FIG. 4A according to various embodiments, the wearable electronic device 200 has been described as including two light emitting units; however, the disclosure is not limited thereto. For example, the wearable electronic device 200 may include more than two light emitting units.

FIG. 4B is a view illustrating a wearable electronic device 200 including a plurality of light emitting units 310 and 415 according to an embodiment of the disclosure.

According to an embodiment, <450> of FIG. 4B is a cross-sectional view illustrating, when viewed from the front, a wearable electronic device (e.g., the wearable electronic device 200 of FIG. 2) including a plurality of light emitting units (e.g., the first light emitting unit 310 and the second light emitting unit 415). According to an embodiment, <470> of FIG. 4B is a view illustrating, when viewed from one side, a wearable electronic device 200 including a plurality of light emitting units (e.g., the first light emitting unit 310 and the second light emitting unit 415).

Referring to <450> and <470> of FIG. 4B, the wearable electronic device 200 may include a plurality of light emitting units (e.g., the first light emitting unit 310 and the second light emitting unit 415). For example, the plurality of light emitting units (e.g., the first light emitting unit 310 and the second light emitting unit 415) may be disposed on a substrate 240 so as to face a specific direction (e.g., to be directed toward the opening 2001). For example, when the wearable electronic device 200 is viewed from the front, the plurality of light emitting units (e.g., the first light emitting unit 310 and the second light emitting unit 415) may be disposed on the substrate 240 so as to overlap each other.

According to an embodiment, the plurality of light emitting units (e.g., a first light emitting unit 310 and a second light emitting unit 415) may have the same wavelength band.

According to an embodiment, as the wearable electronic device 200 includes the plurality of light emitting units (e.g., the first light emitting unit 310 and the second light emitting unit 415), at least one light emitting unit may be used to measure (or acquire) biometric information of a user, and at least another light emitting unit may be used to output visual notification information related to an event.

For example, among the plurality of light emitting units (e.g., the first light emitting unit 310 and the second light emitting unit 415), at least one light emitting unit, for example, the first light emitting unit 310, may measure (or acquire) biometric information of the user by emitting first light 320 under the control of a processor of the wearable electronic device 200 (e.g., the processor 120 of FIG. 1). At least another light emitting unit among the plurality of light emitting units (e.g., the first light emitting unit 310 and the second light emitting unit 415), for example, the second light emitting unit 415, may be electrically connected (e.g., coupled) to the second light guide member 361 and the second light scattering member 363 that are disposed adjacent to the second light emitting unit 415 (e.g., disposed in a second lateral direction, e.g., direction ③, from the second light emitting unit 415). Under the control of the processor of the wearable electronic device 200 (e.g., the processor 120 of FIG. 1), second light 460 emitted through the second light emitting unit 415 may be reflected (or collected) by the electrically connected second light guide member 361. The second light reflected (or collected) by the second light guide member 361 may be transmitted to the outside through the second light scattering member 363, thereby allowing the user to recognize visual notification information related to the event.

According to an embodiment, when the wearable electronic device 200 includes the plurality of light emitting units (e.g., the first light emitting unit 310 and the second light emitting unit 415), a partition member 475 may be disposed between the first light emitting unit 310 and the second light emitting unit 415. As the partition member 475 is disposed between the first light emitting unit 310 and the second light emitting unit 415, interference that may occur between first light 320 emitted through the first light emitting unit 310 and second light 460 emitted through the second light emitting unit 415 may be prevented.

As illustrated in FIGS. 4A and 4B according to various embodiments, the first light 320 emitted through the first light emitting unit 310 and/or the second light 460 emitted through the second light emitting unit 415 may be transmitted, by at least one light guide member (e.g., the first light guide member 341 and/or the second light guide member 361) and at least one light scattering member (e.g., the first light scattering member 343 and/or the second light scattering member 363), in the first lateral direction (e.g., direction ②) and/or the second lateral direction (e.g., direction ③) when the wearable electronic device 200 is viewed from the one side (e.g., direction ①), such that a user may intuitively identify visual notification information related to an event even while wearing the wearable electronic device 200.

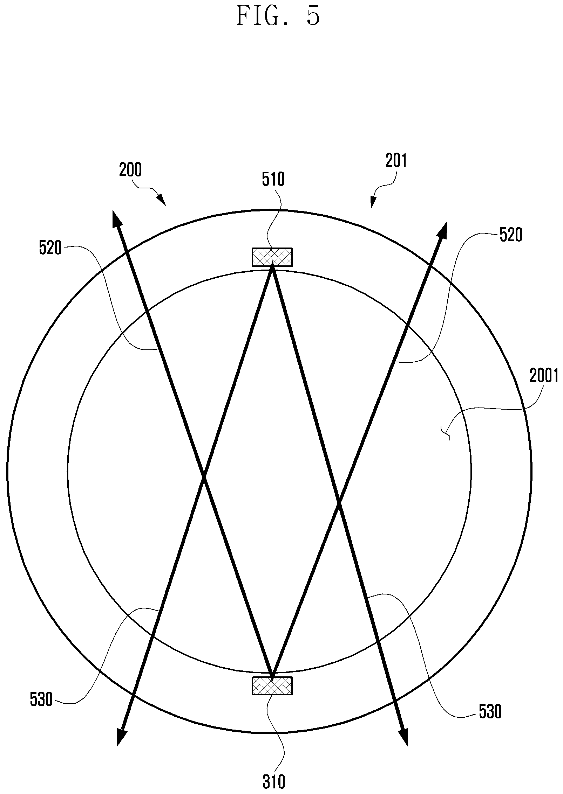

FIG. 5 is a cross-sectional view illustrating, when viewed from the front, a wearable electronic device 200 including a plurality of light emitting units 310 and 510 according to an embodiment of the disclosure.

Referring to FIG. 5, the wearable electronic device (e.g., the wearable electronic device 200 of FIG. 2) may include a plurality of light emitting units (e.g., the first light emitting unit 310 and the second light emitting unit 510). The first light emitting unit 310 and the second light emitting unit 510 may each include a laser diode.

According to an embodiment, the first light emitting unit 310 and the second light emitting unit 510 may be disposed on a substrate (e.g., the substrate 240 of FIG. 2) so as to face a specific direction (e.g., to be directed toward the opening 2001). For example, when the wearable electronic device 200 is viewed from the front, the first light emitting unit 310 and the second light emitting unit 510 may be disposed in an inner space of the housing 201 so as to face each other with the opening 2001 interposed therebetween.

As illustrated in FIG. 5 according to an embodiment, the plurality of light emitting units (e.g., the first light emitting unit 310 and the second light emitting unit 510) are disposed in the inner space of the housing 201 so as to face each other when the wearable electronic device 200 is viewed from the front, and light (e.g., first light 520 and second light 530) emitted through the plurality of light emitting units (e.g., the first light emitting unit 310 and the second light emitting unit 510) is transmitted to the outside by a light guide member (e.g., the first light guide member 341 and/or the second light guide member 361 of FIGS. 4A and 4B) and a light scattering member (e.g., the first light scattering member 343 and/or the second light scattering member 363 of FIGS. 4A and 4B). Therefore, it may be possible to ensure that there are no areas where light is not irradiated. Accordingly, regardless of a direction in which a user wears the wearable electronic device 200, the user may identify visual notification information related to an event through light transmitted to the outside of the wearable electronic device 200 (e.g., in a lateral direction (e.g., direction ② and/or direction ③ of FIGS. 3B and 4B)) (e.g., scattered light (e.g., the scattered light 349 and 367 of FIG. 3B)).

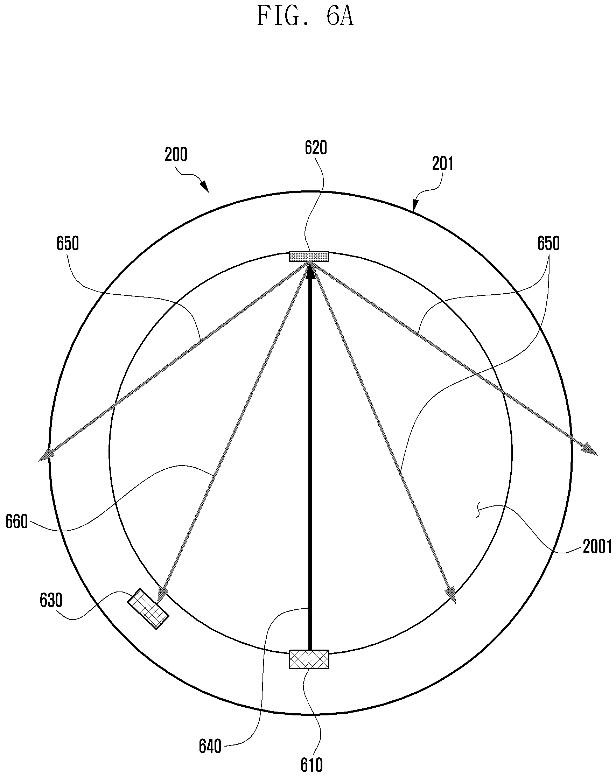

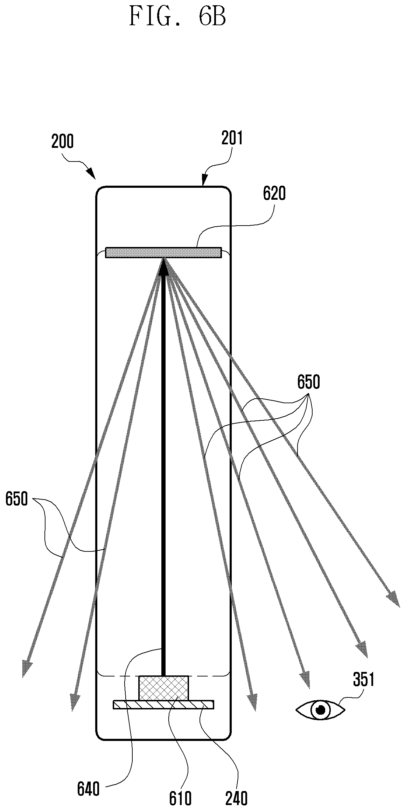

FIG. 6A is a cross sectional view illustrating, when viewed from the front, a wearable electronic device 200 including a light emitting unit 610 according to an embodiment of the disclosure. FIG. 6B is a view illustrating, when viewed from one side, the wearable electronic device 200 including a light emitting unit 610 according to an embodiment of the disclosure.

Referring to FIGS. 6A and 6B, the wearable electronic device (e.g., the wearable electronic device 200 of FIG. 2) may include a light emitting unit 610 (e.g., the light emitting PPG sensors 231-1, 231-2, and 231-3 of FIG. 2).

According to an embodiment, the wearable electronic device 200 may include a substrate 240 disposed in an inner space of a housing 201. For example, the substrate 240 may have flexibility so as to correspond to a curvature of the wearable electronic device 200 (e.g., an inner curvature corresponding to an opening 2001 of the housing 201).

According to an embodiment, the light emitting unit 610 may be disposed on the substrate 240. For example, the light emitting unit 610 may be disposed on the substrate 240 so as to face a specific direction (e.g., to be directed toward the opening 2001).

According to an embodiment, the light emitting unit 610 may include a laser diode.

According to an embodiment, when the light emitting unit 610 includes a laser diode, light 640 emitted through the laser diode may have linearity. Accordingly, the wearable electronic device 200 may include at least one light scattering member for scattering the light 640 having linearity. The at least one light scattering member may be disposed in an inner space of the housing 201 so as to face the light emitting unit 610 with the opening 2001 interposed therebetween when the wearable electronic device 200 is viewed from the front.

According to an embodiment, the at least one light scattering member may include a first light scattering member 620. According to an embodiment, the first light scattering member 620 may include a diffuse reflection member.

For example, the diffuse reflection member may include a grating structure. Light 640 incident on the grating structure may be reflected (650, 660) at a plurality of angles. The plurality of angles may be determined by the grating structure.

As another example, the diffuse reflection member may include a diffusion optics structure (or mirror structure). Light 640 incident on the diffusion optics structure may be reflected (650, 660) in a plurality of directions regardless of a specific angle.

According to an embodiment, light 640 emitted through the light emitting unit 610 may be reflected (650, 660) by the first light scattering member 620 and transmitted to the outside. As the light 640 is reflected (650) by the first light scattering member 620 and transmitted to the outside, the light may be recognized through a user's eye 351.

According to an embodiment, the wearable electronic device 200 may include a light receiving unit 630 configured to receive light that is emitted through the light emitting unit 610 and reflected (660) by the first light scattering member 620.

According to an embodiment, when the diffuse reflection member is formed as a grating structure, the light 640 emitted through the light emitting unit 610 may be reflected (650, 660) at a plurality of angles by the grating structure, and the light receiving unit 630 may be disposed in an inner space of the housing 201 corresponding to one of the plurality of angles.

According to an embodiment, a processor of the wearable electronic device 200 (e.g., the processor 120 of FIG. 1) may identify whether the wearable electronic device 200 is worn on a user's finger based on light received through the light receiving unit 630. For example, based on the light received through the light receiving unit 630, the processor 120 may identify whether the wearable electronic device 200 is worn on the user's finger through an annular opening 2001 at the center of the wearable electronic device 200 or whether a charging cradle is inserted through the annular opening 2001.

According to an embodiment, when light emitted through the light emitting unit 610 is back-scattered by an object (e.g., a finger or a charging cradle) present in the annular opening 2001 at the center of the wearable electronic device 200 and is received by the light receiving unit 630, the processor 120 may identify that the wearable electronic device 200 is in a state of being worn on a user's finger.

According to an embodiment, when the light 640 emitted through the light emitting unit 610 is not received by the light receiving unit 630, the processor 120 may identify that the wearable electronic device 200 is in a state in which a charging cradle is inserted through the annular opening 2001 at the center of the wearable electronic device 200. For example, the charging cradle may be made of plastic, and accordingly, the light 640 emitted through the light emitting unit 610 may not be transmitted to or pass through the charging cradle. In other words, when the charging cradle is inserted through the annular opening 2001 at the center of the wearable electronic device 200, back scattering may not occur unlike the case where the wearable device 200 is worn on the user's finger described above. Therefore, there may be no light received by the light receiving unit 630. Based on this, when the light 640 emitted through the light emitting unit 610 is not received by the light receiving unit 630, the processor 120 may identify that the wearable electronic device 200 is in a state in which the charging cradle is inserted through the annular opening 2001 at the center of the wearable electronic device 200.

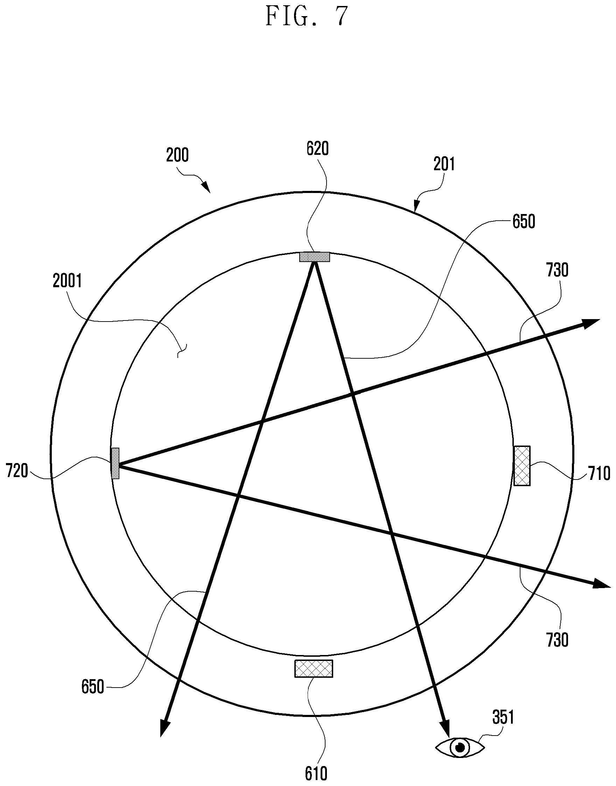

In FIGS. 6A and 6B according to various embodiments, the wearable electronic device 200 has been described as including a single light emitting unit (e.g., the light emitting unit 610); however, the disclosure is not limited thereto. For example, the wearable electronic device 200 may include a plurality of light emitting units. In this regard, various embodiments will be described below with reference to FIG. 7.

FIG. 7 is a cross-sectional view illustrating, when viewed from the front, a wearable electronic device 200 including a plurality of light emitting units 610 and 710 according to an embodiment of the disclosure.

Referring to FIG. 7, the wearable electronic device (e.g., the wearable electronic device 200 of FIG. 2) may include a plurality of light emitting units, for example, a first light emitting unit 610 and a second light emitting unit 710 (e.g., the light emitting PPG sensors 231-1, 231-2, and 231-3 of FIG. 2). For example, the first light emitting unit 610 and the second light emitting unit 710 may be disposed on a substrate (e.g., the substrate 240 of FIG. 2) so as to face a specific direction (e.g., to be directed toward the opening 2001).

According to an embodiment, the first light emitting unit 610 and the second light emitting unit 710 may each include a laser diode.

According to an embodiment, the wearable electronic device 200 may include a first light scattering member 620 configured to scatter first light emitted through the first light emitting unit 610 and a second light scattering member 720 configured to scatter second light emitted through the second light emitting unit 710.

According to an embodiment, when the wearable electronic device 200 is viewed from the front, the first light scattering member 620 may be disposed in an inner space of the housing 201 so as to face the first light emitting unit 610 with the opening 2001 interposed therebetween. When the wearable electronic device 200 is viewed from the front, the second light scattering member 720 may be disposed in the inner space of the housing 201 so as to face the second light emitting unit 710 with the opening 2001 interposed therebetween.

As illustrated in FIG. 7 according to an embodiment, the wearable electronic device 200 includes a plurality of light emitting units (e.g., the first light emitting unit 610 and the second light emitting unit 710) and a plurality of light scattering members (e.g., the first light scattering member 620 and the second light scattering member 720) configured to scatter a plurality of light beams emitted through the plurality of light emitting units (e.g., the first light emitting unit 610 and the second light emitting unit 710), such that light 650 and 730 reflected by the plurality of light scattering members (e.g., the first light scattering member 620 and the second light scattering member 720) is transmitted to the outside. Therefore, it may be possible to ensure that there are no areas where light is not irradiated. Accordingly, regardless of a direction in which a user wears the wearable electronic device 200, the user may identify visual notification information related to an event through the reflected light 650 and 730.

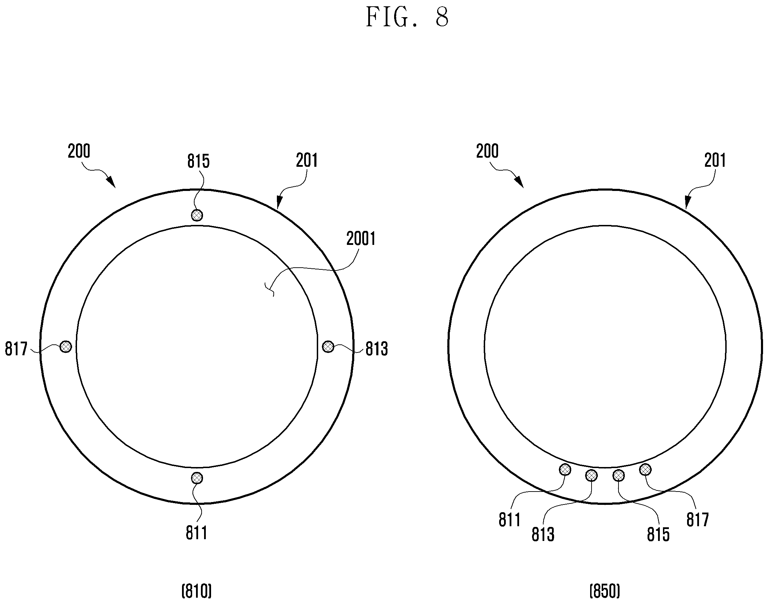

FIG. 8 is a view illustrating modes in which a plurality of light emitting units 811, 813, 815, and 817 are disposed in a wearable electronic device 200 according to an embodiment of the disclosure.

Referring to <810> of FIG. 8 according to an embodiment, a housing 201 of a wearable electronic device (e.g., the wearable electronic device 200 of FIG. 2) may be formed in an annular shape.

According to an embodiment, the wearable electronic device 200 may include a plurality of light emitting units (e.g., the first light emitting unit 811, the second light emitting unit 813, the third light emitting unit 815, and/or the fourth light emitting unit 817).

According to an embodiment, the first light emitting unit 811, the second light emitting unit 813, the third light emitting unit 815, and/or the fourth light emitting unit 817 may be disposed on at least a portion of a substrate (e.g., the substrate 240 of FIG. 2) disposed in an inner space of the housing 201, along a shape of the housing 201, for example, the annular shape. For example, the first light emitting unit 811, the second light emitting unit 813, the third light emitting unit 815, and/or the fourth light emitting unit 817 may be disposed on at least a portion of the substrate 240 so as to correspond to each other with an opening 2001 interposed therebetween, along the annular shape of the housing 201. For example, the first light emitting unit 811 and the third light emitting unit 815 may be disposed on at least a portion of the substrate 240 so as to correspond to each other with the opening 2001 interposed therebetween. The second light emitting unit 813 and the fourth light emitting unit 817 may be disposed on at least a portion of the substrate 240 so as to correspond to each other with the opening 2001 interposed therebetween.

The disclosure is not limited thereto. Referring to <850> of FIG. 8 according to an embodiment, the first light emitting unit 811, the second light emitting unit 813, the third light emitting unit 815, and/or the fourth light emitting unit 817 may be disposed on at least a portion of a substrate 240 disposed in an inner space of the housing 201 corresponding to one side region of the wearable electronic device 200 (e.g., a lower region when the wearable electronic device 200 is viewed from the front).

According to the embodiments described above with reference to FIGS. 2 to 8, a processor of the wearable electronic device 200 (e.g., the processor 120 of FIG. 1) may emit light through at least one light emitting unit when an occurrence of an event is detected.

According to an embodiment, the event may include an event for emitting light through the at least one light emitting unit. For example, the event may include information related to measurement (or acquisition) of biometric information of the wearable electronic device 200, information related to pairing with an external electronic device (e.g., the electronic device 102 or the electronic device 104 of FIG. 1), and/or state information of the wearable electronic device 200.

According to an embodiment, the information related to measurement (or acquisition) of biometric information of the wearable electronic device 200 may include state information related to a biometric sensor (e.g., the PPG photoplethysmogram sensors 231-1, 231-2, 231-3, 232-1, and 232-2 of FIG. 2). For example, the information related to measurement (or acquisition) of biometric information of the wearable electronic device 200 may include information indicating a state in which biometric information is being measured (or acquired) through the biometric sensor and/or biometric information acquired through the biometric sensor.

According to an embodiment, state information of the wearable electronic device 200 may include temperature information inside the wearable electronic device 200, external humidity information, remaining capacity information of a battery (e.g., the battery 189 of FIG. 1), state information related to wearing of the wearable electronic device 200, state information related to charging of the wearable electronic device 200, and/or state information related to a location of the wearable electronic device 200.

According to an embodiment, the processor 120 may control brightness, color, or emission state of light emitted through at least one light emitting unit, and/or the number of light emitting units that emit light, so as to correspond to an occurred event. The processor 120 may cause light to be emitted through the at least one light emitting unit based on the controlled brightness, color, emission state, and the number of light emitting units that emit light.

For example, as described above with reference to FIG. 8, when the wearable electronic device 200 includes a plurality of light emitting units (e.g., the first light emitting unit 811, the second light emitting unit 813, the third light emitting unit 815, and the fourth light emitting unit 817), the processor 120 may control the first light emitting unit 811, the second light emitting unit 813, the third light emitting unit 815, and the fourth light emitting unit 817 to sequentially emit light. As the first light emitting unit 811, the second light emitting unit 813, the third light emitting unit 815, and the fourth light emitting unit 817 sequentially emit light, the light may be emitted in a form such as rotating in a specific direction (e.g., leftward or rightward).

As another example, the processor 120 may identify remaining capacity information of the battery 189 and may cause light to be emitted through at least some of the plurality of light emitting units so as to correspond to the remaining capacity information of the battery 189.

As another example, as described above with reference to FIGS. 6A and 6B, the processor 120 may identify, based on light received through a light receiving unit (e.g., the light receiving unit 630 of FIG. 6A), whether the wearable electronic device 200 is worn on a user's finger through an annular opening (e.g., the opening 2001 of FIG. 2) at the center of the wearable electronic device 200, or whether a charging cradle is inserted through the annular opening. The processor 120 may allow light corresponding to the state (e.g., a state in which the wearable electronic device 200 is worn on a user's finger or a state in which the wearable electronic device 200 is fitted onto a charging cradle) to be emitted through at least one light emitting unit.

As another example, an external electronic device communicatively connected to the wearable electronic device 200 may execute a function for finding the wearable electronic device 200. In this case, the processor 120 of the wearable electronic device 200 may allow light to be emitted through at least one light emitting unit based on a signal received from the communicatively connected external electronic device (e.g., a signal related to execution of the function for finding the wearable electronic device 200).

In various embodiments, based on detection of an occurrence of the above described event, the processor 120 may emit light through at least one light emitting unit, and the light may be transmitted to the outside by a light guide member (e.g., the first light guide member 341 and/or the second light guide member 361 of FIGS. 3A and 3B) and/or a light scattering member (e.g., the first light scattering member 343 and/or the second light scattering member 363 of FIGS. 3A and 3B, or the first light scattering member 620 and/or the second light scattering member 720 of FIGS. 6 and 7), so as to allow the user to recognize the light. Accordingly, the user may more intuitively identify visual notification information related to the event through the light.

According to an embodiment, the processor 120 may provide auditory notification information through an audio module (e.g., the audio module 170 of FIG. 1) disposed inside the wearable electronic device 200 and/or tactile notification information through a haptic module (e.g., the haptic module 179 of FIG. 1), together with visual notification information based on light emitted through at least one light emitting unit.

According to an embodiment of the disclosure, a wearable electronic device 101 or 200 may include a housing 201 and a substrate 240 disposed in an inner space of the housing 201. According to an embodiment, the wearable electronic device 101 or 200 may include at least one light emitting unit 310 disposed on the substrate 240. According to an embodiment, when the wearable electronic device 101 or 200 is viewed from one side, the wearable electronic device 101 or 200 may include at least one light guide member 341 or 361 disposed adjacent to the at least one light-emitting unit 310. According to an embodiment, the wearable electronic device 101 or 200 may include at least one light-scattering member 343 or 363 disposed such that light emitted through the at least one light-emitting unit 310 and reflected by the at least one light guide member 341 or 361 is transmitted to the outside.

According to an embodiment, the at least one light guide member 341 or 361 may include a total reflection film optical fiber.

According to an embodiment, the at least one light scattering member 343 or 363 may include at least one light diffusion film.

According to an embodiment, the at least one light emitting unit 310 may include a light emitting diode LED.

According to an embodiment, the at least one light guide member 341 or 361 may include a first light guide member 341. According to an embodiment, when the wearable electronic device 101 or 200 is viewed from one side, the first light guide member 341 may be disposed adjacent to the at least one light emitting unit 310 in a direction of a first lateral surface of the at least one light emitting unit 310.

According to an embodiment, the at least one light guide member 341 or 361 may further include a second light guide member 361. According to an embodiment, when the wearable electronic device 101 or 200 is viewed from one side, the second light guide member 361 may be disposed adjacent to the at least one light emitting unit 310 in a direction of a second lateral surface of the at least one light emitting unit 310.

According to an embodiment, the at least one light scattering member 343 or 363 may include a first light scattering member 343. According to an embodiment, when the wearable electronic device 101 or 200 is viewed from one side, the first light scattering member 343 may be disposed on at least a portion of the housing 201 in a direction of a first lateral surface of the first light guide member 341.

According to an embodiment, the at least one light scattering member 343 or 363 may further include a second light scattering member 363. According to an embodiment, when the wearable electronic device 101, 200 is viewed from one side, the second light scattering member 363 may be disposed on at least a portion of the housing 201 in a direction of a second lateral surface of the second light guide member 361.

According to an embodiment, the at least one light emitting unit 310 may include a plurality of light emitting units 310 and 415.

According to an embodiment, the plurality of light emitting units 310 and 415 may be disposed adjacent to each other.

According to an embodiment, the plurality of light emitting units 310 and 415 may have different wavelength bands or the same wavelength band.

According to an embodiment, when the plurality of light emitting units 310 and 415 have the same wavelength band, at least one light emitting unit among the plurality of light emitting units 310 and 415 may be used to measure biometric information.

According to an embodiment, when the plurality of light emitting units 310 and 415 have the same wavelength band, at least another one of the plurality of light emitting units 310 and 415 may be electrically connected to the at least one light guide member 341 or 361 and the at least one light scattering member 343 or 363, so as to allow light emitted through at least one other light emitting unit and reflected by the at least one light guide member 341, 361 to be transmitted to the outside through the at least one light scattering member 343 or 363.

According to an embodiment, the housing 201 of the wearable electronic device 101 or 200 may include an annular opening 2001 at a center thereof. According to an embodiment, the wearable electronic device 101 or 200 may be worn on a user's finger through the opening 2001.

According to an embodiment, the wearable electronic device 101 or 200 may further include a processor 120 disposed on the substrate 240. According to an embodiment, the processor 120 may control the at least one light emitting unit 310 to emit light when an occurrence of an event is detected.

According to an embodiment, the processor 120 may control at least one of a color, a brightness, an emission state, and the number of light emitting units that emit light, based on the detected event, with respect to light emitted through the at least one light emitting unit 310.

According to an embodiment of the disclosure, a wearable electronic device 101 or 200 may include a housing 201 and a substrate 240 disposed in an inner space of the housing 201. According to an embodiment, the wearable electronic device 101 or 200 may include at least one light emitting unit 610 disposed on the substrate 240. According to an embodiment, when the wearable electronic device 101 or 200 is viewed from the front, the wearable electronic device 101 or 200 may include at least one light scattering member 620 disposed in the inner space of the housing 201 so as to face the at least one light emitting unit 610. According to an embodiment, light emitted through the at least one light emitting unit 610 may be reflected by the at least one light scattering member 620 and transmitted to the outside.

According to an embodiment, the at least one light scattering member 620 may include at least one diffuse reflection member.

According to an embodiment, the at least one light emitting unit 610 may include at least one laser diode.

According to an embodiment, the wearable electronic device 101 or 200 may further include at least one light receiving unit 630 configured to receive light reflected by the at least one light scattering member 620.

According to an embodiment, the wearable electronic device 101 or 200 may further include a processor 120 disposed on the substrate 240. According to an embodiment, the processor 120 may be configured to identify whether the wearable electronic device 101 or 200 is worn on a user's finger, based on light received via the at least one light receiving unit 630.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively," as "coupled with," "coupled to," "connected with," or "connected to" another element (e.g., a second element), it means that the element may be coupled with the other element directly (e.g., through wires), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device (101, 200) comprising:
a housing (201);
a substrate (240) disposed in an internal space of the housing (201);
at least one light emitting unit (310) disposed on the substrate (240);
at least one light guide member (341, 361) disposed adjacent to the at least one light emitting unit (310), respectively, when the wearable electronic device (101, 200) is viewed from one side; and
at least one light scattering member (343, 363) disposed such that light emitted by the at least one light emitting unit (310) to be reflected by the at least one light guide member (341, 361) is transmitted to the outside.

2. The wearable electronic device (101, 200) of claim 1, wherein the at least one light guide member (341, 361) comprises a total reflection film optical fiber.

3. The wearable electronic device (101, 200) of claim 1 or 2, wherein the at least one light scattering member (343, 363) comprises at least one light diffusion film.

4. The wearable electronic device (101, 200) of any of claims 1 to 3, wherein the at least one light emitting unit (310) comprises a light emitting diode (LED).

5. The wearable electronic device (101, 200) of any of claims 1 to 4, wherein the at least one light guide member (341, 361) comprises at least one of a first light guide member (341) or a second light guide member (361),
wherein the first light guide member (341) is disposed adjacent to the at least one light emitting unit (310) in a direction of a first lateral surface of the at least one light emitting unit (310), when the wearable electronic device (101, 200) is viewed from the one side, and
wherein the second light guide member (361) is disposed adjacent to the at least one light emitting unit (310) in a direction of a second lateral surface of the at least one light emitting unit (310), when the wearable electronic device (101, 200) is viewed from the one side.

6. The wearable electronic device (101, 200) of claim 5, wherein the at least one light scattering member (343, 363) comprises at least one of a first light scattering member (343) or a second light scattering member (363),
wherein the first light scattering member (343) is disposed in at least a part of the housing (201) in a direction of a first lateral surface of the first light guide member (341), when the wearable electronic device (101, 200) is viewed from the one side, and
wherein the second light scattering member (363) is disposed in at least a part of the housing in a direction of a second lateral surface of the second light guide member (361), when the wearable electronic device (101, 200) is viewed from the one side.

7. The wearable electronic device (101, 200) of any of claims 1 to 6, wherein the at least one light emitting unit (310) comprises a plurality of light emitting units (310, 415),
wherein the plurality of light emitting units (310, 415) are arranged adjacent to each other, and
wherein the plurality of light emitting units (310, 415) have different wavelength bands or a same wavelength band.

8. The wearable electronic device (101, 200) of claim 7, wherein, when the plurality of light emitting units (310, 415) have the same wavelength band, at least one light emitting unit among the plurality of light emitting units (310, 415) is used to measure biometric information.

9. The wearable electronic device (101, 200) of claim 8, wherein, when the plurality of light emitting units (310, 415) have the same wavelength bands, at least one other light emitting unit among the plurality of light emitting units (310, 415) is electrically connected to the at least one light guide member (341, 361) and the at least one light scattering member (343, 363) such that a light emitted by the at least one other light emitting unit to be reflected by the at least one light guide member (341, 361) is transmitted to the outside via the at least one light scattering member (343, 363).

10. The wearable electronic device (101, 200) of any of claims 1 to 9, wherein the housing (201) comprises an annular opening (2001) in the center, and
wherein the wearable electronic device (101, 200) is worn on the user's finger through the opening (2001).

11. The wearable electronic device (101, 200) of any of claims 1 to 10, further comprising:
a processor (120) disposed on the substrate (240),
wherein the processor (120) is configured to control, when detecting an occurrence of an event, the at least one light emitting unit (310) to emit a light.

12. The wearable electronic device (101, 200) of claim 11, wherein the processor (120) is further configured to control at least one of a color, brightness, an emission state of light emitted through the at least one light emitting unit (310), or the number of light emitting unit emitting the light, based on the detected event.

13. A wearable electronic device (101, 200) comprising:
a housing (201);
a substrate (240) disposed in an internal space of the housing (201);
at least one light emitting unit (610) disposed on the substrate (240); and
at least one light scattering member (620) disposed in the inner space of the housing (201) to face the at least one light emitting unit (610), when the wearable electronic device (101, 200) is viewed from a front side,
wherein light emitted by the at least one light emitting unit (610) is reflected by the at least one light scattering member (620) to be transmitted to the outside.

14. The wearable electronic device (101, 200) of claim 13, wherein the at least one light scattering member (620) comprises at least one diffuse reflection member, and
wherein the at least one light emitting unit (610) comprises at least one laser diode.

15. The wearable electronic device (101, 200) of claim 13 or 14, further comprising:
at least one light receiving unit (630) configured to receive light reflected by the at least one light scattering member (620); and
a processor (120) disposed on the substrate (240),
wherein the processor (120) is configured to identify whether the wearable electronic device (101, 200) is worn on the user's finger, based on light received via the at least one light receiving unit (630).
